(19)

Europäisches
Patentamt
European
Patent Office
Office européen
des brevets

(11) **EP 4 295 766 B1**

(12) **EUROPEAN PATENT SPECIFICATION**

(45) Date of publication and mention
of the grant of the patent:
**23.04.2025 Bulletin 2025/17**

(21) Application number: **23180614.2**

(22) Date of filing: **21.06.2023**

(51) International Patent Classification (IPC):
***A61B 5/12*** *(2006.01)*

(52) Cooperative Patent Classification (CPC):
**A61B 5/126**

(54) **AIR-PUMP ARRANGEMENT FOR AN ACOUSTIC MEASUREMENT DEVICE**

LUFTPUMPENANORDNUNG FÜR EIN AKUSTISCHES MESSGERÄT

ENSEMBLE POMPE À AIR POUR UN DISPOSITIF DE MESURE ACOUSTIQUE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **23.06.2022 EP 22180714**

(43) Date of publication of application:
**27.12.2023 Bulletin 2023/52**

(73) Proprietor: **Interacoustics A/S
5500 Middelfart (DK)**

(72) Inventors:
• **NØRGAARD, Kren Monrad
5500 Middelfart (DK)**
• **SJURSEN, Jonas
5500 Middelfart (DK)**

(74) Representative: **Demant
Demant A/S
Kongebakken 9
2765 Smørum (DK)**

(56) References cited:
**WO-A1-2021/149036      DE-A1- 102005 017 091
US-A1- 2006 197 412      US-A1- 2015 342 504
US-A1- 2016 220 155**

**Description**

**FIELD**

The present application relates to an acoustic measurement device configured to provide acoustic property values for objective ear-condition evaluation of a patient's ear, wherein at least part of the acoustic measurement device being configured to be positioned within an ear canal of the patient and configured to take a resting position within the ear canal. The acoustic measurement device comprises an ear probe and the acoustic measurement device comprises an air-pump arrangement. The air-pump arrangement, at least in the resting position, being in fluid connection with the ear canal through at least one tube running via the ear probe. The air-pump arrangement, in the resting position, is configured to apply an air-pressure control procedure, consisting of at least one air-pressure level, across the patient's tympanic membrane of the ear, wherein the acoustic measurement device, in the resting position, being configured to provide acoustic property values in reaction to the air-pressure control procedure applied by the pump arrangement across the patient's tympanic membrane of the ear.

The application further relates to a diagnostic instrumental arrangement configured to provide at least one objective ear-condition parameter of a patient's ear comprising an acoustic measurement device according to the application, further comprising an acoustic input unit configured to capture acoustic signals representative for the air-pressure levels within the ear canal, a control device configured to control the pump arrangement, and a processing device configured to further process the captured acoustic signals to an evaluation device, the evaluation device being configured to execute an evaluation process to the processed acoustic signals associated to the air-pressure control procedure to obtain the at least one objective ear-condition parameter for the patient's ear.

Moreover, the present application relates to an objective ear-condition evaluation method using a diagnostic instrumental arrangement according to the application.

**BACKGROUND**

Acoustic measurement devices and diagnostic instrumental arrangements are known in the art for example from tympanometric diagnostic methods, diagnostic methods for pressurized acoustic-reflex measurement, or diagnostic methods for otoacoustic-emission measurement.

Those instruments known in the art for hearing diagnostics conventionally utilize piston, peristaltic, or gear pumps. These pumps are characterized by being driven by an electric motor, and operating the motor at some constant power generates a constant volume-displacement rate.

These pumps offer suitable performance when it comes to perform air-pressure control procedures as they are for example standardized for several diagnostic type instruments in IEC 60645-5 (2005), thereby operating-even in the case of tympanometry-within a rather small pressure-difference region (with magnitudes up to 600 daPa) relative to the absolute ambient pressure (approximately 10132.5 daPa), resulting in an approximately constant-rate pressure sweep usually requiring no or little regulation.

However, these pumps have several less-desirable properties such as their mechanical complexity, their large size, and their relatively slow responsiveness due to the internal mass which needs to move before the pressure changes. In particular, as regards the application of a piston pump, the respective acoustic measurement device then further suffers from its inherent displacement limits.

US2016220155A1 relates to an audiologic test apparatus including a pump device configured to apply a first pressure to the ear canal; and a processing module for communicatively coupling to the pump device and to a signal generator, wherein the processing module is configured to obtain first acoustic parameter values indicative of an acoustic parameter at the first pressure based on a first broadband signal generated using the signal generator; wherein the pump device is configured to change the first pressure to a changed pressure, and wherein the pump device is also configured to apply a second pressure to the ear canal; and wherein the processing module is also configured to obtain second acoustic parameter values indicative of the acoustic parameter at the second pressure based on a second broadband signal generated using the signal generator, and determine a middle ear resonance frequency based on the first and second acoustic parameter values.

US2015342504A1 relates to an audiologic test apparatus including a processing unit configured to conduct a first and second audiologic tests; a first probe connector for connecting a first probe for the first audiologic test; a second probe connector for connecting a second probe for the second audiologic test; and a pump module connected to a first fluid port of the first probe connector with a first fluid channel and to a second fluid port of the second probe connector with a second fluid channel, wherein the pump module in a first operating mode is in fluid communication with the first fluid port, and in a second operating mode is in fluid communication with the second fluid port, the pump module configured to modify pressure in the first fluid channel when in the first operating mode, and to modify pressure in the second fluid channel when in the second operating mode.

Therefore, there is a need to provide a solution that addresses at least some of the above-mentioned challenges that come along with the known acoustic measurement devices and their air-pump arrangement.

## SUMMARY

Thus, it is the object of the present application to provide an acoustic measurement device and a diagnostic instrumental arrangement as described above, which do not show the disadvantages described above, or at least show them to a lesser extent, and which, in particular, provide a space saving design for an acoustic measurement device, which reduces the overall effort, e.g. in terms of handling the acoustic measurement device and instrumental arrangement during diagnostics, and facilitates simple production of such device, however at the same time providing reliable results for the objective ear-condition evaluation of the patient's ear.

The above objects are achieved starting from an acoustic measurement device according to the preamble of claim 1 by the features of the characterizing part of claim 1. The above objects are further achieved by the diagnostic instrumental arrangement according to claim 14 and are also achieved by a method of using the acoustic measurement device according to claim 16.

The present application is based on the technical teaching that a more space saving design results, for example, in a more handy operation of the respective instrument. Further, reliable results for objective ear-condition evaluation can be accomplished, if instead of the known pump configurations applied in the field of objective acoustic measurements so far (e.g. piston, peristaltic, or gear pumps), a membrane pump (also known as a diaphragm pump) configuration is used. The membrane pump configuration may (preferably) be formed as a piezo-electric membrane pump configuration (e.g. a miniature piezo-electric membrane pump configuration).

This alternative type of pump, namely a membrane pump and in particular in the form of a miniature piezo-electric membrane pump, generally operates by displacing an elastic element, in particular a membrane, in a harmonic motion.

Usually, in its regular field of application, this pump then relies on internal check valves (i.e., allowing flow in one but not the other direction) to turn this oscillating displacement into an air flow, which enters through an inlet during the first half cycle and exits through the outlet in the second half cycle. Thus, these kind of membrane pumps are known to be inherently unidirectional, a directionality which is defined by the orientation of the check valves.

Hence, according to one aspect, the present application relates to an acoustic measurement device configured to provide acoustic property values for objective ear-condition evaluation of a patient's ear, in particular a human's ear, wherein at least part of said acoustic measurement device being configured to be positioned within an ear canal of said patient and configured to take a resting position within said ear canal, said acoustic measurement device comprising an ear probe, said acoustic measurement device comprising an air-pump arrangement, said air-pump arrangement, at least in said resting position, being in fluid connection with said ear canal through at least one tube running via said ear probe, said air-pump arrangement, in said resting position, being configured to apply an air-pressure control procedure, consisting of at least one air-pressure level, across said patient's tympanic membrane of said ear, wherein said acoustic measurement device, in said resting position, being configured to provide acoustic property values in reaction to said air pressure control procedure applied by said pump arrangement across said patient's tympanic membrane of said ear.

Said air-pump arrangement comprising at least two pumps, wherein said at least two pumps comprising a movable element in the form of a membrane.

For example, the provided acoustic property values comprise values representative for acoustic impedance against said patient's tympanic membrane of said ear.

For example, the provided acoustic property values comprise values representative for acoustic admittance against said patient's tympanic membrane of said ear.

For example, the provided acoustic property values comprise values representative for absorbance.

Then, the acoustic measurement device may act as a tympanometer and a tympanogram can be obtained.

For example, the provided acoustic property values comprise values representative for otoacoustic emission.

The acoustic measurement device according to the application is especially suitable for providing acoustic property values for objective ear-condition evaluation for human's ears, but the application is not limited thereto. The concept of the device and the instrument according to the application could be applied for objective ear-condition evaluation of animals as well.

Basically, the membrane of each of the pumps of the pump arrangement can be formed in any suitable way. Very beneficial configurations can be achieved in terms of compactness and size-reduction of the pump arrangement if the at least two pumps are configured to act as membrane pumps for applying the air-pressure control procedure and particularly if they are formed as membrane pumps. Their miniature size allows an easier handling of the device and consequently an easier handling of the entire instrument according to the application.

In preferred variants of the application, the acoustic measurement device further comprises an acoustic input unit that is configured to capture acoustic signals representative for the air-pressure levels within the ear canal. The acoustic measurement device may comprise a control device that is configured to control the air pump arrangement. For example, the control device may comprise any form of regulation algorithm/controller to control the air-pump arrangement. For example, the control device may comprise a proportional-integral-derivative (PID) controller to control the air-pump arrangement, in particular using a regulation algorithm to perform for example constant pressure-sweep rates. This is particularly beneficial when the acoustic measurement device is used in tympanometry. The regulation algorithm may be based on an air pressure measured by a pressure sensor close to the acoustic input unit. The acoustic measurement device may further comprise a processing device that is configured to further process the captured acoustic signals.

In the invention, the at least two pumps are formed as piezo-electric membrane pumps. The piezo-electric membrane pumps are then each arranged fluidically in series with at least one flow-regulating component, wherein they are acting fluidically in parallel. Therein, each of the flow regulating components may be configured and arranged within the air pump arrangement such that at least one piezo-electric membrane pump is capable of working bidirectional when applying the air-pressure control procedure.

Therein, in very beneficial variants of the application, it is provided that the at least two pumps comprise a first pump and a second pump, where the first pump and the second pump being poled in opposing directions to each other.

So, in other words, the air pump arrangement may be configured such that the at least two pumps, preferably being (miniature) piezo-electric membrane pumps, are capable of working simultaneously to control the air pressure level within the air pressure control procedure in the ear canal.

The flow regulating components in the above-described configurations may be formed as flow resistors.

In some preferred variants of the application, an air pump arrangement provides a first piezo-electric membrane pump fluidically in series with a first flow resistor and a second piezo-electric membrane pump fluidically in series with a second flow resistor. The air pump arrangement may be configured and arranged such that pressure between the first flow resistor and said second flow resistor can be controlled in the ear-canal of the respective ear. Thereby, the air pump arrangement may essentially act in the manner analogous to a voltage divider, by maintaining and adjusting an inlet pressure and an outlet pressure at the first and the second piezo-electric membrane pump.

Further preferred variants of the application comprise an acoustic muffler that is provided and arranged at the ear probe. For example, the acoustic muffler may attenuate acoustic noise generated by the air-pump arrangement from entering the ear canal and interfering with ongoing acoustic measurement. The acoustic muffler may be configured to constitute the flow regulating components described above, preferably in the form of a flow resistor. For example, the acoustic muffler may consist of a network of narrow tubes and cavities, configured to attenuate acoustic noise and acting in a manner of a flow resistor.

In certain preferred variants of the application, (for example, when the acoustic measurement device is used for tympanometry, and hence acts in the form of a tympanometer), its air pump arrangement, in the resting position within the patient's ear, is configured to apply the air pressure control procedure in the form of maintaining essentially a single air pressure level during the procedure. Conventionally, the air pressure level lies in the range of -600 to +400 daPa.

In addition, or as an alternative, the air pump arrangement, in the resting position, may be configured to apply the air pressure control procedure in the form of performing varying air pressure levels during the respective procedure with varying air pressure levels during the procedure ranging between around -600 daPa to +400 daPa. In such a case, preferably, the respective procedure follows the standardized procedure of varying air pressure for different instrumental types according to IEC 60645-5 (2005). For example, the air pump arrangement, in the resting position, may be configured to perform these varying air-pressure levels with an adjustable air-pressure sweep rate. Thereby, a tympanogram can be obtained in a reliable manner but with a more handy and compact acoustic measurement device thanks to the use of membrane pumps, in particular in the form of miniature piezo-electric membrane pumps.

The acoustic measurement device may be configured to provide acoustic property values representative for the patient's middle-ear in reaction to a first pressure control procedure of the air pressure control procedure, applied by the at least two pumps of said air pump arrangement across the patient's tympanic membrane of the ear.

The first pressure control procedure may contain performing varying air pressure levels during the procedure ranging between around -600 daPa to +400 daPa. Therein, the provided acoustic property values may be values representative for acoustic impedance against the patient's tympanic membrane of the respective ear to be diagnosed. In such a configuration for tympanometry, the air pump arrangement, in the resting position, may be configured to perform the varying air-pressure levels with an adjustable air pressure sweep rate. Preferably, also here, the air pump arrangement is capable of performing the air pressure sweep rates following the standardized procedure according to IEC 60645-5 (2005).

In addition, or as an alternative, the acoustic measurement device may be configured to provide acoustic property values representative for the patient's inner ear in reaction to a second pressure-control procedure of the air-pressure control procedure, applied by the at least two pumps of the pump arrangement across the patient's tympanic membrane of the ear.

The second pressure control procedure may contain performing and maintaining essentially a single air-pressure level during the procedure. The essentially single air pressure level performed may lie in the range of -600 to +400 daPa. The acoustic measurement device may be configured to act as a hearing diagnostic instrument for pressurized acoustic-reflex measurement.

In addition, or as an alternative, the acoustic measurement device may be configured to provide acoustic property values representative for the patient's inner-ear in reaction to a third pressure control procedure of the air-pressure control procedure, applied by the at least two pumps of the pump arrangement across the patient's tympanic membrane of the ear. The third pressure control procedure may contain performing and maintaining essentially a single air pressure level during the procedure. The essentially single air pressure level performed may lie in the range of -600 to +400 daPa. The acoustic measurement device may be configured to act as hearing diagnostic instrument for otoacoustic-emission measurement.

According to a further aspect, the present application relates to a diagnostic instrumental arrangement that is configured to provide at least one objective ear-condition parameter of a patient's ear, in particular of a human's ear. The diagnostic instrumental arrangement comprises an acoustic measurement device according to any of the configurations or variants according to the application as described above. Furthermore, an acoustic input unit is provided and configured to capture acoustic signals representative for the air-pressure levels within the ear canal. Further, a control device is provided that is configured to control the pump arrangement according to any of the configurations or variants according to the application as described above. Finally, the diagnostic instrumental arrangement comprises a processing device configured to further process the captured acoustic signals to an evaluation device, the evaluation device being configured to execute an evaluation process to the processed acoustic signals associated to the air-pressure control procedure to obtain the at least one objective ear-condition parameter the said patient's ear.

The diagnostic instrumental arrangement may be is mechanically connectable to a stand-alone device and/or may be operated via a mobile end device, in particular a tablet or a smartphone.

According to a further aspect, the present application relates to an objective ear-condition evaluation method which uses a diagnostic instrumental arrangement as described above with an acoustic measurement device according to any one of the configurations or variants of the application as described above.

It is contemplated that the acoustic measurement device may comprise said air-pump arrangement, which comprises at least one pump (such as only one pump), wherein said at least one pump comprising a movable element in the form of a membrane. Further, said air-pump arrangement may comprise at least one flow regulating component, such as one or two flow regulating components.

Further preferred variants of the present application will become apparent from the dependent claims and the following description of a preferred embodiment of the acoustic measurement device and instrument arrangement which refers to the appended figures.

## BRIEF DESCRIPTION OF THE DRAWINGS

FIG. 1 shows an exemplary acoustic measurement device according to the application.

FIG. 2 shows a typical pressure-flow relationship of a typical membrane pump operated at its maximum power.

FIG. 3 shows a schematic illustration of an example according to the application of an electrical-analog circuit of two membrane pumps, represented by sources, connected to the volume of the ear-canal, represented by a capacitor, each in series with a flow resistor. Ground represents ambient pressure.

FIG. 4 shows a schematic illustration of an example according to the application of producing a tympanometric air-pressure procedure using two membrane pumps in terms of the ear-canal pressure (top), the pressure rate (middle), and the relative pump power of the two pumps (bottom).

## DETAILED DESCRIPTION OF THE APPLICATION

In the following, an example of an acoustic measurement device **1** is shown and described, which comprises an exemplary air pump arrangement **2** according to the application. The air pump arrangement 2 may comprise a first and a second pump. The first and/or the second pump may be a membrane pump. For example, the first and/or the second pump may be a piezo-electric membrane pump. For example, the first and/or the second pump may be a miniature piezo-electric membrane pump. The acoustic measurement device **1** may be used in a diagnostic instrumental arrangement (not further shown here).

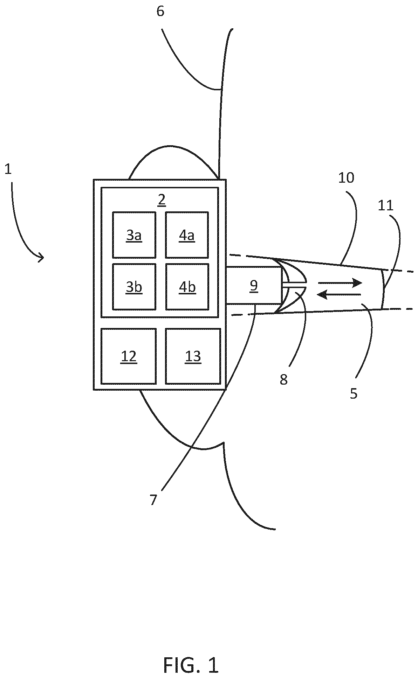

In FIG. 1, it is shown that the air-pump arrangement **2** of the acoustic measurement device **1** provides a first pump **3a (e.g. a** miniature piezo-electric membrane pump) fluidically in series with a first flow resistor **4a** and a second pump **3b** (e.g. a miniature piezo-electric membrane pump) fluidically in series with a second flow resistor **4b.** The air-pump arrangement **2** is configured and arranged such that pressure between the first flow resistor **4a** and said second flow resistor **4b** can be controlled in the ear-canal of the respective ear. Thereby, the air pump arrangement **2** essentially acts in the manner analogous to a voltage divider, by maintaining and adjusting an inlet pressure and an outlet pressure at the first and the second pump **3a, 3b.**

Further, in FIG. 1, it is shown that at least part of said acoustic measurement device **1** may be positioned within an ear canal 5 of a patient 6 and be configured to take a resting position within said ear canal 5. The acoustic measurement device **1** may comprise an ear probe 7 positioned at least partly within said ear canal 5.

The ear probe 7 may comprise an ear tip 8, which may be releasably attached to an ear-probe body 9. When the ear probe 7 has been inserted into the ear canal 5 (and take a resting position), the ear tip 8 may provide a barometric seal toward the ear-canal walls 10 of the ear canal 5. The ear probe 7 may provide a stimulus into the ear of the patient 6 in a direction towards the eardrum 11 and receive a reflected part of said stimulus (i.e. capture acoustic signals), as indicated by the two arrows located in the ear canal 5.

The acoustic measurement device 1 may further comprise a control device 12 configured to control said pump arrangement 2.

The acoustic measurement device **1** may further comprise a processing device 13 configured to process said captured acoustic signals. An evaluation device of said processing device 13 may be configured to execute an evaluation process to said processed acoustic signals associated to an air-pressure control procedure to obtain at least one objective ear-condition parameter for said patient's 6 ear.

Preferably, the acoustic measurement device **1** is used for tympanometry, hence acts in the form of a tympanometer. However, in addition, the acoustic measurement device **1** may also be configured to perform diagnostic methods for pressurized acoustic-reflex measurement or diagnostic methods for otoacoustic-emission measurement.

The air pump arrangement **2** may be configured to apply an air pressure control procedure in the form of maintaining essentially a single air pressure level during the procedure. For example, the air pressure level then lies in the range of -600 to +400 daPa.

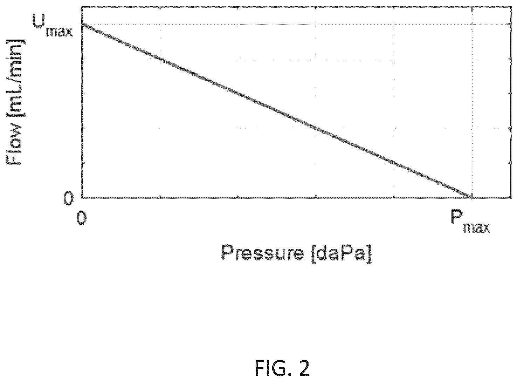

In addition, the air pump arrangement **2** may be configured to apply the air pressure control procedure in the form of performing varying air pressure levels during the respective procedure with varying air pressure levels during the procedure in the range of around -600 daPa to +400 daPa. The air pump arrangement **2** may be capable of performing the standardized procedures of varying air pressure for different instrumental types according to IEC 60645-5 (2005), which will be described by way of an example in the context with FIG. 2 to 4 below.

In FIG. 2, a typical relationship between maximum pressure $P_{(max)}$ and maximum flow $U_{max}$ of a single membrane pump (herein **3a** or **3b**), as known in the art operated at maximum power is illustrated. Decreasing the power delivered to the pump results in an offset parallel to this linear relationship.

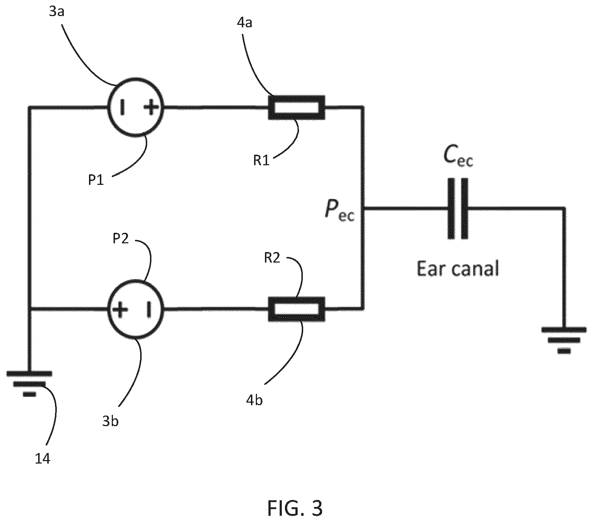

FIG. 3 illustrates the operating principle of the air pump arrangement **2** of the acoustic measurement device **1** by an electrical-analog circuit of the two membrane pumps **3a, 3b,** represented by sources, connected to the volume of the ear-canal, represented by a capacitor $C_{ec}$, each in series with a flow regulating component in the form of the flow resistor **4a, 4b.** The ground 14 represents ambient pressure.

Unlike a regular membrane-pump arrangement where two membrane pumps, each one being fluidically in series with one check valve, never operate simultaneously, the air pump arrangement **2,** as given in FIG. 3, is based on simultaneous operation of the two membrane pumps formed as membrane pumps **3a, 3b.**

While this configuration of air-pump arrangement **2** restricts the total flow through the system (compared to the case of no flow resistors), it allows accurately controlling the pressure between the two flow resistors **4a, 4b,** i.e., in the ear canal, in a manner analogous to a voltage divider, by maintaining and adjusting the pressure at the outlet and inlet of the membrane pumps **3a** and **3b,** respectively and, thus, the flow through the resistors **4a, 4b.** That is, maintaining a constant air pressure in the ear canal requires continuous operation of one membrane pump (**3a** or **3b**).

This is opposed to the prior art pumps used in acoustic measurement devices, as piston pumps, peristaltic pumps, and gear pumps, where maintaining a constant ear-canal pressure simply requires stopping the pump.

In particular, the air pump arrangement **2** solves the issue associated with regular membrane pumps of not being able to control the flow through the membrane pump **3a, 3b** due to a negative pressure drop in the flow direction because the pressure drops in the system occur across the two flow resistors **4a, 4b.** That is, each membrane pump **3a, 3b** can generate a change in pressure between the membrane pump **3a, 3b** and flow resistor **4a, 4b,** regardless of the pressure in the ear canal, and force a larger flow through the resistor than dictated by the ear-canal-to-ambient pressure difference.

Depending on the flow resistances $R_1$ (associated with flow resistor **4a)** and $R_2$ (associated with flow resistor **4b),** operating membrane pumps **3a** and **3b** at some constant power results in a given point of operation on the pressure-flow relationship in FIG. 2 and the pressures $P_1$ and $P_2$. Disregarding non-linear effects by assuming that the flow resistances $R_1$ and $R_2$ of each flow resistor **4a** and **4b** does not depend on the flow, the pressure in the ear canal $P_{ec}$ when the system is in a steady state can be calculated based on the voltage-divider equation,

$$P_{ec} = \frac{(P_1 - P_2)R_2}{R_1 + R_2}.$$

Thus, manipulating **P₁** and **P₂** by controlling the drive power to each membrane pump **3a, 3b** any pressure can be achieved in the ear canal **Pₑc** and this pressure can be altered in any direction.

Deriving the pump drive powers that yield a constant-rate ear-canal pressure $P_{ec}(t)$ is more complicated because the pressure-flow operational point of each pump changes temporarily in response to sudden changes in power until a steady state has been reattained. Assuming that the pressure-flow operational point remains constant and that a change in pump powers result in proportional changes in **P₁(t) or P₂(t),** the ear-canal pressure response $P_{ec}(t)$ resulting from step changes in pump drive powers takes the form of an exponential decay,

$$P_{ec}(t) = a\,e^{-bt} + c.$$

The constants a, b, and c are mathematical expressions comprised by **R₁, R₂,** the compliance of the ear canal **Cₑc** (which is inversely proportional to its volume), and the initial and final values of the pressure step functions **P₁(t)** and **P₂(t).** Consequently, the pressures **P₁(t)** or **P₂(t)** required to produce linear changes to the ear-canal pressure **Pₑc(t)** when operating only one membrane pump **3a** or **3b** at a time can be immediately calculated.

While it is possible to predict how the membrane pumps **3a, 3b** should be driven to achieve ear-canal pressure with constant pressure sweep rates with the given approximations and a given ear-canal volume, practical application in ear-canals of varying volumes require a regulation algorithm. In such a case, for example, a PID controller is used to achieve these constant pressure-sweep rates. For example, this regulation algorithm is based on the air pressure measured by a pressure sensor close to the ear probe.

Accordingly, the acoustic measurement device may comprise a pressure sensor.

A standard air-pressure procedure required to generate a tympanogram may usually consists of three principal steps.

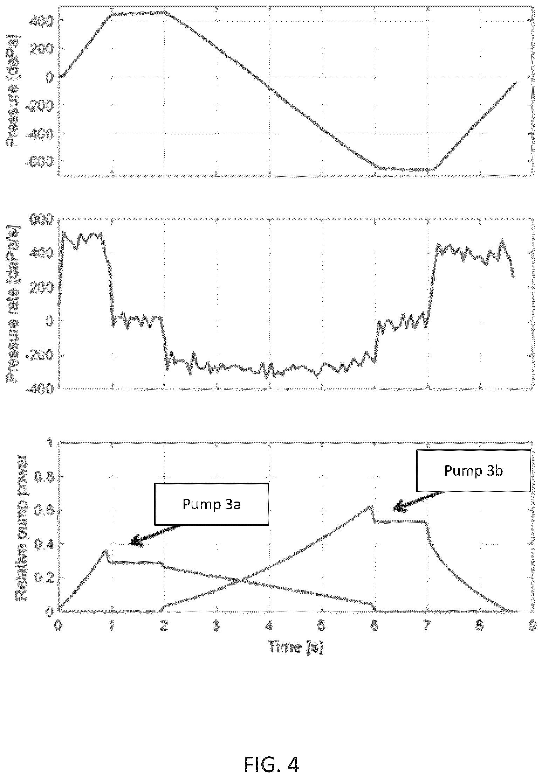

First, the air pump arrangement is used to pump to a desired starting pressure. In terms of the pressure difference across the tympanic membrane, this starting pressure may be within at least the range of -600 to +200 daPa for type-1 instruments and -200 to +200 daPa for type-2 and type-3 instruments (IEC 60645-5 (2005)), although instruments often support wider pressure ranges, e.g., -600 to +400 daPa (see FIG. 4).

The second step involves a pressure sweep, specified by a constant pressure sweep rate in units of daPa/s (positive or negative depending on the direction of the sweep), from the starting pressure to the stopping pressure of opposite operational sign. Type-1 instruments must be capable of maintaining a constant sweep rate of 50 daPa/s with a tolerance of ±10 daPa/s for the duration of the sweep (IEC 60645-5 (2005)), passing the 0-daPa pressure-difference point. The acoustic measurements of the middle ear may be acquired during this pressure sweep of the second step.

Finally, as the third step, the ear-canal pressure is returned to ambient pressure in a controlled manner to minimize patient discomfort, concluding the pressurization procedure. Other air-pressure procedures used in tympanometry may include a manual tympanogram, where the operator adjusts the pressure in the ear canal using, e.g., a slider, while observing the acoustic changes to the middle ear.

FIG. 4 now exemplifies the application of the acoustic measurement device **1** with two membrane pumps **3a, 3b** and the two (precision-orifice) flow resistors **4a, 4b** to produce the above described standardized 3 step sequence of air pressure procedure of a tympanogram in a small cavity in terms of the pressure measured by the pressure sensor (top), the pressure rate (middle), and the relative pump drive power of the two membrane pumps **3a, 3b** (bottom).

The sequence shown in FIG. 4 is not based on a regulation algorithm; instead, pump drive-power curves were predefined with trial-and-error adjustments to produce approximately linear pressure changes in the given cavity volume. It is evident how only membrane pump **3a** is required for the initial linear pressurization of the ear canal, and only membrane pump **3b** is required for the concluding linear depressurization. Notice also the simultaneous operation of the two membrane pumps **3a, 3b** during the pressure sweep, crossing the 0-daPa point. For the pressure sweep in this example, the power of pump **3a** was set to linearly decrease over some specified time while the power of pump **3b** was adjusted to create a linear pressure sweep.

**REFERENCES**

IEC 60645-5 (2005)    IEC 60645-5, 2005 *"Instruments for the measurement of aural acoustic impedance/admittance. (International Electrotechnical Commission, Geneva, Switzerland) "*

# EP 4 295 766 B1

**Claims**

1.  An acoustic measurement device (1) configured to provide acoustic property values for objective ear-condition evaluation of a patient's ear, wherein

    - at least part of said acoustic measurement device (1) being configured to be positioned within an ear canal of said patient and configured to take a resting position within said ear canal,
    - said acoustic measurement device (1) comprising an ear probe,
    - said acoustic measurement device (1) comprising an air-pump arrangement (2), said air-pump arrangement (2), at least in said resting position, being in fluid connection with said ear canal through at least one tube running via said ear probe,
    - said air-pump arrangement (2), in said resting position, being configured to apply an air-pressure control procedure, consisting of at least one air-pressure level, across said patient's tympanic membrane of said ear, wherein
    - said acoustic measurement device (1), in said resting position, being configured to provide acoustic property values in reaction to said air pressure control procedure applied by said air-pump arrangement (2) across said patient's tympanic membrane of said ear,

    **characterized in that**,

    - said air-pump arrangement (2) comprising at least two pumps (3a, 3b), wherein said at least two pumps are formed as piezo-electric membrane pumps and are each arranged fluidically in series with at least one flow-regulating component and are configured to act fluidically in parallel.

2.  Acoustic measurement device (1) according to claim 1, wherein said at least two pumps (3a, 3b) are configured to act as membrane pumps (3a, 3b) for applying said air-pressure control procedure.

3.  Acoustic measurement device (1) according one of the preceding claims, comprising

    - an acoustic input unit configured to capture acoustic signals representative for said air-pressure levels within said ear canal,
    - a control device configured to control said air-pump arrangement (2), and
    - a processing device configured to further process said captured acoustic signals.

4.  Acoustic measurement device (1) according to any one of the preceding claims, wherein,

    - said at least one flow regulating component being configured and arranged within said air-pump arrangement (2) such that said two piezo-electric membrane pumps (3a, 3b) is capable of working bidirectional when applying said air-pressure control procedure.

5.  Acoustic measurement device (1) according to any one of the preceding claims, wherein

    - said at least two pumps (3a, 3b) comprising a first pump (3a) and a second pump (3b), wherein said first pump (3a) and said second pump (3b) being poled in opposing directions to each other, and/or
    - said at least two pumps (3a, 3b) are formed as piezo-electric membrane pumps (3a, 3b), and/or
    - said air-pump arrangement (2) being configured such that said at least two pumps (3a, 3b), preferably piezo-electric membrane pumps (3a, 3b), can work simultaneously to control said air pressure level within said air pressure control procedure in said ear canal.

6.  Acoustic measurement device (1) according to claim 5, wherein

    - said at least two pumps (3a, 3b) are formed as piezo-electric membrane pumps (3a, 3b) which are each fluidically arranged in series with at least one flow regulating component (4a, 4b), in particular in the form of a flow resistor (4a, 4b), wherein the two piezo-electric membrane pumps (3a, 3b) are arranged parallel to each other.

7.  Acoustic measurement device (1) according to claim 6, wherein

- said air-pump arrangement (2) providing a first piezo-electric membrane pump (3a) fluidically in series with a first flow resistor (4a) and a second piezo-electric membrane pump (3b) fluidically in series with a second flow resistor (4b), wherein said first piezo-electric membrane pump (3a) and said second piezo-electric membrane pump (3b) are arranged parallel to each other,
wherein
- said air-pump arrangement (2) being configured and arranged such that pressure between said first flow resistor (4a) and said second flow resistor (4b) can be controlled in said ear-canal of said ear,
- said air-pump arrangement (2) thereby in particular essentially acting in a manner analogous to a voltage divider, by maintaining and adjusting an inlet pressure and an outlet pressure at said first (3a) and said second piezo-electric membrane pump (3b).

8. Acoustic measurement device (1) according to any one of claims 3 to 7, wherein

- an acoustic muffler is provided and arranged at said ear probe,
- said acoustic muffler being configured to constitute said flow regulating components, in particular in the form of a flow resistor, and configured to attenuate noise from said air-pump arrangement (2),
- said acoustic muffler in particular consisting of a network of narrow tubes and cavities, configured to attenuate acoustic noise and acting in a manner of a flow resistor.

9. Acoustic measurement device (1) according to any one of the preceding claims, wherein

- said air-pump arrangement (2), in said resting position, being configured to apply said air pressure control procedure in the form of maintaining essentially a single air pressure level during said procedure, wherein
- said air pressure level lying in the range of -600 to +400 daPa,
and/or

- said air-pump arrangement (2), in said resting position, being configured to apply said air pressure control procedure in the form of performing varying air pressure levels during said procedure, wherein said varying air pressure levels during said procedure range between around -600 daPa to +400 daPa, wherein
- in particular, said air-pump arrangement (2), in said resting position, being configured to perform said varying air-pressure levels with an adjustable air-pressure sweep rate.

10. Acoustic measurement device (1) according to any one of the preceding claims, wherein

- said acoustic measurement device (1) being configured to provide acoustic property values representative for said patient's middle-ear in reaction to a first pressure control procedure of said air pressure control procedure, applied by said at least two pumps (3a, 3b) of said air-pump arrangement (2) across said patient's tympanic membrane of said ear, wherein
- said first pressure control procedure in particular contains performing varying air pressure levels during said procedure, wherein said varying air pressure levels during said first pressure control procedure range between around -600 daPa to +400 daPa, wherein
- in particular, said air-pump arrangement (2), in said resting position, being configured to perform said varying air-pressure levels with an adjustable air pressure sweep rate.
- said acoustic measurement device (1) in particular being configured to act as a tympanometer.

11. Acoustic measurement device (1) according to any one of the preceding claims, wherein

- said acoustic measurement device (1) being configured to provide acoustic property values representative for said patient's inner ear in reaction to a second pressure control procedure of said air pressure control procedure, applied by said at least two pumps of said air-pump arrangement (2) across said patient's tympanic membrane of said ear, wherein
- said second pressure control procedure in particular contains performing and maintaining essentially a single air pressure level during said procedure, wherein
- said essentially single air pressure level performed in particular lying in the range of -600 to +400 daPa,
- said acoustic measurement device (1) in particular being configured to act as a hearing diagnostic instrument for pressurized acoustic-reflex measurement.

12. Acoustic measurement device (1) according to any one of the preceding claims, wherein

- said acoustic measurement device (1) being configured to provide acoustic property values representative for said patient's inner-ear in reaction to a third pressure control procedure of said air-pressure control procedure, applied by said at least two pumps (3a, 3b) of said air-pump arrangement (2) across said patient's tympanic membrane of said ear, wherein
- said third pressure control procedure in particular contains performing and maintaining essentially a single air pressure level during said procedure, wherein
- said essentially single air pressure level performed in particular lying in the range of -600 to +400 daPa,
- said acoustic measurement device (1) in particular being configured to act as a hearing diagnostic instrument for otoacoustic-emission measurement.

13. Acoustic measurement device (1) according to any one of the preceding claims, wherein the acoustic measurement device comprises a control device configured to control said air-pump arrangement (2).

14. A diagnostic instrumental arrangement configured to provide at least one objective ear-condition parameter of a patient's ear, in particular of a human's ear, comprising

- an acoustic measurement device (1) according to any one of claims 1 to 13,
- a processing device configured to further process said captured acoustic signals to an evaluation device, said evaluation device being configured to execute an evaluation process to said processed acoustic signals associated to said air-pressure control procedure to obtain said at least one objective ear-condition parameter for said patient's ear.

15. A diagnostic instrumental arrangement according to claim 14, wherein said diagnostic instrumental arrangement being mechanically connectable to a stand-alone device and/or being operated via a mobile end device, in particular a tablet or a smartphone.

16. Method using an acoustic measurement device (1) according to any one of claims 1-13 and/or a diagnostic instrumental arrangement according to any one of claims 14 or 15, the method comprising

- positioning at least part of the acoustic measurement device (1) within an ear canal of a patient to take a resting position within said ear canal,
- providing a fluid connection between an air-pump arrangement (2) of said acoustic measurement device (1), at least in said resting position, and said ear canal through at least one tube running via an ear probe of said acoustic measurement device (1),
- applying an air-pressure control procedure, consisting of at least one air-pressure level, across said patient's tympanic membrane of said ear, by the air-pump arrangement (2), in said resting position, and
- providing acoustic property values in reaction to said air pressure control procedure applied by said air-pump arrangement (2) across said patient's tympanic membrane of said ear, by said acoustic measurement device (1), in said resting position.

**Patentansprüche**

1. Akustische Messvorrichtung (1), die dazu konfiguriert ist, akustische Eigenschaftswerte für die objektive Beurteilung des Ohrzustands eines Ohrs eines Patienten bereitzustellen, wobei

- mindestens ein Teil der akustischen Messvorrichtung (1) dazu konfiguriert ist, innerhalb eines Gehörgangs des Patienten positioniert zu sein, und dazu konfiguriert ist, eine Ruheposition innerhalb des Gehörgangs einzunehmen,
- die akustische Messvorrichtung (1) eine Ohrsonde umfasst,
- die akustische Messvorrichtung (1) eine Luftpumpenanordnung (2) umfasst, wobei die Luftpumpenanordnung (2) mindestens in der Ruheposition in Fluidverbindung mit dem Gehörgang durch zumindest eine Röhre steht, die über die Ohrsonde verläuft,
- die Luftpumpenanordnung (2) in der Ruheposition dazu konfiguriert ist, eine Luftdrucksteuerungsprozedur, bestehend aus mindestens einem Luftdruckpegel, über das Trommelfell des Ohrs des Patienten anzuwenden, wobei
- die akustische Messvorrichtung (1) in der Ruheposition dazu konfiguriert ist, akustische Eigenschaftswerte als Reaktion auf die Luftdrucksteuerprozedur bereitzustellen, der durch die Luftpumpenanordnung (2) über das Trommelfell des Ohres des Patienten angewendet wird,

**dadurch gekennzeichnet, dass**

- die Luftpumpenanordnung (2) mindestens zwei Pumpen (3a, 3b) umfasst, wobei die mindestens zwei Pumpen als piezoelektrische Membranpumpen ausgebildet sind und jeweils fluidisch in Reihe mit mindestens einer strömungsregulierenden Komponente angeordnet und dazu konfiguriert sind, fluidisch parallel zu wirken.

2. Akustische Messvorrichtung (1) nach Anspruch 1, wobei die mindestens zwei Pumpen (3a, 3b) dazu konfiguriert sind, als Membranpumpen (3a, 3b) zum Anwenden der Luftdrucksteuerprozedur zu wirken.

3. Akustische Messvorrichtung (1) nach einem der vorhergehenden Ansprüche, umfassend:

   - eine akustische Eingabeeinheit, die dazu konfiguriert ist, akustische Signale aufzunehmen, die für die Luftdruckpegel innerhalb des Gehörgangs repräsentativ sind,
   - eine Steuervorrichtung, die dazu konfiguriert ist, die Luftpumpenanordnung (2) zu steuern, und
   - eine Verarbeitungsvorrichtung, die dazu konfiguriert ist, die erfassten akustischen Signale weiter zu verarbeiten.

4. Akustische Messvorrichtung (1) nach einem der vorhergehenden Ansprüche, wobei

   - die mindestens eine strömungsregulierende Komponente derart innerhalb der Luftpumpenanordnung (2) konfiguriert und angeordnet ist, dass die zwei piezoelektrischen Membranpumpen (3a, 3b) dazu in der Lage sind, bidirektional zu arbeiten, wenn die Luftdrucksteuerprozedur angewendet wird.

5. Akustische Messvorrichtung (1) nach einem der vorhergehenden Ansprüche, wobei

   - die mindestens zwei Pumpen (3a, 3b) eine erste Pumpe (3a) und eine zweite Pumpe (3b) umfassen, wobei die erste Pumpe (3a) und die zweite Pumpe (3b) in zueinander entgegengesetzten Richtungen gepolt sind, und/oder
   - die mindestens zwei Pumpen (3a, 3b) als piezoelektrische Membranpumpen (3a, 3b) ausgebildet sind und/oder
   - die Luftpumpenanordnung (2) derart konfiguriert ist, dass die mindestens zwei Pumpen (3a, 3b), vorzugsweise piezoelektrische Membranpumpen (3a, 3b), simultan arbeiten können, um den Luftdruckpegel innerhalb der Luftdrucksteuerprozedur in dem Gehörgang zu steuern.

6. Akustische Messvorrichtung (1) nach Anspruch 5, wobei

   - die mindestens zwei Pumpen (3a, 3b) als piezoelektrische Membranpumpen (3a, 3b) ausgebildet sind, die jeweils mit mindestens einer strömungsregulierenden Komponente (4a, 4b) fluidisch in Reihe angeordnet sind, insbesondere in Form eines Strömungswiderstands (4a, 4b), wobei die zwei piezoelektrischen Membranpumpen (3a, 3b) parallel zueinander angeordnet sind.

7. Akustische Messvorrichtung (1) nach Anspruch 6, wobei

   - die Luftpumpenanordnung (2) eine erste piezoelektrische Membranpumpe (3a), die fluidisch in Reihe mit einem ersten Strömungswiderstand (4a) angeordnet ist, und eine zweite piezoelektrische Membranpumpe (3b), die fluidisch in Reihe mit einem zweiten Strömungswiderstand (4b) angeordnet ist, bereitstellt, wobei die erste piezoelektrische Membranpumpe (3a) und die zweite piezoelektrische Membranpumpe (3b) parallel zueinander angeordnet sind,
   wobei
   - die Luftpumpenanordnung (2) derart konfiguriert und angeordnet ist, dass der Druck zwischen dem ersten Strömungswiderstand (4a) und dem zweiten Strömungswiderstand (4b) in dem Gehörgang des Ohres gesteuert werden kann,
   - die Luftpumpenanordnung (2) dadurch insbesondere im Wesentlichen auf eine zu einem Spannungsteiler analoge Weise wirkt, indem ein Einlassdruck und ein Auslassdruck an der ersten (3a) und der zweiten piezoelektrischen Membranpumpe (3b) aufrechterhalten und eingestellt werden.

8. Akustische Messvorrichtung (1) nach einem der Ansprüche 3 bis 7, wobei

   - ein Schalldämpfer bereitgestellt und an der Ohrsonde angeordnet ist,
   - der Schalldämpfer dazu konfiguriert ist, die strömungsregulierenden Komponenten zu bilden, insbesondere in der Form eines Strömungswiderstands, und dazu konfiguriert ist, Geräusche von der Luftpumpenanordnung (2) zu dämpfen,
   - der Schalldämpfer, der insbesondere aus einem Netzwerk aus schmalen Röhren und Hohlräumen besteht, dazu konfiguriert ist, Schallgeräusche zu dämpfen, und auf eine Weise eines Strömungswiderstands wirkt.

9. Akustische Messvorrichtung (1) nach einem der vorhergehenden Ansprüche, wobei

   - die Luftpumpenanordnung (2) in der Ruheposition dazu konfiguriert ist, die Luftdrucksteuerprozedur in der Form anzuwenden, dass während der Prozedur im Wesentlichen ein einzelner Luftdruckpegel aufrechterhalten wird, wobei
   - der Luftdruckpegel im Bereich von -600 bis +400 daPa liegt,
   und/oder
   - die Luftpumpenanordnung (2) in der Ruheposition dazu konfiguriert ist, die Luftdrucksteuerprozedur in der Form des Durchführens variierender Luftdruckpegel während des Vorgangs anzuwenden, wobei die variierenden Luftdruckpegel während des Vorgangs zwischen etwa -600 daPa und +400 daPa liegen, wobei
   - insbesondere die Luftpumpenanordnung (2) in der Ruheposition dazu konfiguriert ist, die variierenden Luftdruckpegel mit einer einstellbaren Luftdruckschwungrate durchzuführen.

10. Akustische Messvorrichtung (1) nach einem der vorhergehenden Ansprüche, wobei

   - die akustische Messvorrichtung (1) dazu konfiguriert ist, akustische Eigenschaftswerte, die für das Mittelohr des Patienten repräsentativ sind, als Reaktion auf eine erste Drucksteuerprozedur der Luftdrucksteuerprozedur bereitzustellen, die durch die mindestens zwei Pumpen (3A, 3B) der Luftpumpenanordnung (2) über das Trommelfell des Ohrs des Patienten angewendet wird, wobei
   - die erste Drucksteuerprozedur insbesondere Durchführen variierender Luftdruckpegel während der Prozedur enthält, wobei die variierenden Luftdruckpegel während der ersten Druckregelungsprozedur zwischen etwa -600 daPa bis +400 daPa liegen, wobei
   - insbesondere die Luftpumpenanordnung (2) in der Ruheposition dazu konfiguriert ist, die variierenden Luftdruckpegel mit einer einstellbaren Luftdruckschwungrate durchzuführen,
   - die akustische Messvorrichtung (1) insbesondere dazu konfiguriert ist, als ein Tympanometer zu fungieren.

11. Akustische Messvorrichtung (1) nach einem der vorhergehenden Ansprüche, wobei

   - die akustische Messvorrichtung (1) dazu konfiguriert ist, akustische Eigenschaftswerte, die für das Innenohr des Patienten repräsentativ sind, als Reaktion auf eine zweite Drucksteuerprozedur der Luftdrucksteuerprozedur bereitzustellen, die durch die mindestens zwei Pumpen der Luftpumpenanordnung (2) über das Trommelfell des Ohrs des Patienten angewendet wird, wobei
   - die zweite Druckregelungsprozedur insbesondere Durchführen und Aufrechterhalten im Wesentlichen eines einzelnen Luftdruckpegels während der Prozedur umfasst, wobei
   - der im Wesentlichen einzelne Luftdruckpegel insbesondere im Bereich von -600 bis +400 daPa liegt,
   - die akustische Messvorrichtung (1) insbesondere dazu konfiguriert ist, als ein Hördiagnoseinstrument für eine unter Druck stehende akustische Reflexmessung zu fungieren.

12. Akustische Messvorrichtung (1) nach einem der vorhergehenden Ansprüche, wobei

   - die akustische Messvorrichtung (1) dazu konfiguriert ist, akustische Eigenschaftswerte, die für das Innenohr des Patienten repräsentativ sind, als Reaktion auf eine dritte Drucksteuerprozedur der Luftdrucksteuerprozedur bereitzustellen, die durch die mindestens zwei Pumpen (3A, 3B) der Luftpumpenanordnung (2) über das Trommelfell des Ohrs des Patienten angewendet wird, wobei
   - die dritte Druckregelungsprozedur insbesondere Durchführen und Aufrechterhalten im Wesentlichen eines einzelnen Luftdruckpegels während der Prozedur umfasst, wobei
   - der im Wesentlichen einzelne Luftdruckpegel insbesondere im Bereich von -600 bis +400 daPa liegt,
   - die akustische Messvorrichtung (1) insbesondere dazu konfiguriert ist, als ein Hördiagnostikinstrument für die otoakustische Emissionsmessung zu fungieren.

**13.** Akustische Messvorrichtung (1) nach einem der vorhergehenden Ansprüche, wobei die akustische Messvorrichtung eine Steuervorrichtung umfasst, die dazu konfiguriert ist, die Luftpumpenanordnung (2) zu steuern.

**14.** Diagnoseinstrumentenanordnung, die dazu konfiguriert ist, mindestens einen objektiven Ohrzustandsparameter eines Ohrs eines Patienten, insbesondere eines menschlichen Ohrs, bereitzustellen, umfassend

- eine akustische Messvorrichtung (1) nach einem der Ansprüche 1 bis 13,
- eine Verarbeitungsvorrichtung, die dazu konfiguriert ist, die aufgenommenen Schallsignale weiter an eine Auswertungsvorrichtung zu verarbeiten, wobei die Auswertungsvorrichtung dazu konfiguriert ist, einen Bewertungsprozess zu den verarbeiteten Schallsignalen auszuführen, die mit der Luftdrucksteuerprozedur assoziiert sind, um den mindestens einen objektiven Ohrzustandsparameter für das Ohr des Patienten zu erhalten.

**15.** Diagnoseinstrumentenanordnung nach Anspruch 14, wobei die Diagnoseinstrumentenanordnung mechanisch mit einer eigenständigen Vorrichtung verbindbar ist und/oder über eine mobile Endvorrichtung, insbesondere ein Tablet oder ein Smartphone, betrieben wird.

**16.** Verfahren unter Verwendung einer akustischen Messvorrichtung (1) nach einem der Ansprüche 1-13 und/oder einer Diagnoseinstrumentenanordnung nach einem der Ansprüche 14 oder 15, wobei das Verfahren Folgendes umfasst:

- Positionieren mindestens eines Teils der akustischen Messvorrichtung (1) innerhalb eines Gehörgangs eines Patienten, um eine Ruheposition innerhalb des Gehörgangs einzunehmen,
- Bereitstellen einer Fluidverbindung zwischen einer Luftpumpenanordnung (2) der akustischen Messvorrichtung (1), zumindest in der Ruheposition, und dem Gehörgang durch mindestens eine Röhre, die über eine Ohrsonde der akustischen Messvorrichtung (1) verläuft,
- Anwenden einer Luftdrucksteuerprozedur, die aus mindestens einem Luftdruckpegel besteht, über das Trommelfell des Ohres des Patienten durch die Luftpumpenanordnung (2) in der Ruheposition und
- Bereitstellen von Werten für akustische Eigenschaften als Reaktion auf die Luftdrucksteuerprozedur, die durch die Luftpumpenanordnung (2) über das Trommelfell des Ohrs des Patienten durch die akustische Messvorrichtung (1) in der Ruheposition angewendet wird.

**Revendications**

**1.** Dispositif de mesure acoustique (1) conçu pour fournir des valeurs de propriétés acoustiques pour une évaluation objective de l'état de l'oreille d'un patient, dans lequel

- au moins une partie dudit dispositif de mesure acoustique (1) étant conçue pour être positionnée à l'intérieur d'un conduit auditif dudit patient et conçue pour prendre une position de repos à l'intérieur dudit conduit auditif,
- ledit dispositif de mesure acoustique (1) comprenant une sonde auriculaire,
- ledit dispositif de mesure acoustique (1) comprenant un ensemble de pompe à air (2), ledit ensemble de pompe à air (2), au moins dans ladite position de repos, étant en connexion fluidique avec ledit conduit auditif par l'intermédiaire d'au moins un tube passant par ladite sonde auriculaire,
- ledit ensemble de pompe à air (2), dans ladite position de repos, étant conçu pour appliquer une procédure de commande de pression d'air, constituée d'au moins un niveau de pression d'air, à travers la membrane tympanique de ladite oreille dudit patient, dans lequel
- ledit dispositif de mesure acoustique (1), dans ladite position de repos, étant conçu pour fournir des valeurs de propriétés acoustiques en réaction à ladite procédure de commande de pression d'air appliquée par ledit ensemble de pompe à air (2) à travers la membrane tympanique de ladite oreille dudit patient,

**caractérisé en ce que**,

- ledit ensemble de pompe à air (2) comprenant au moins deux pompes (3a, 3b), dans lequel lesdites au moins deux pompes sont formées comme des pompes à membrane piézo-électriques et étant chacune agencées fluidiquement en série avec au moins un composant de régulation d'écoulement et étant conçues pour agir fluidiquement en parallèle.

**2.** Dispositif de mesure acoustique (1) selon la revendication 1, dans lequel lesdites au moins deux pompes (3a, 3b) sont conçues pour agir comme des pompes à membrane (3a, 3b) pour appliquer ladite procédure de commande de pression d'air.

3. Dispositif de mesure acoustique (1) selon l'une des revendications précédentes, comprenant

   - une unité d'entrée acoustique conçue pour capturer des signaux acoustiques représentatifs desdits niveaux de pression d'air à l'intérieur dudit conduit auditif,
   - un dispositif de commande conçu pour commander ledit ensemble de pompe à air (2), et
   - un dispositif de traitement conçu pour traiter davantage lesdits signaux acoustiques capturés.

4. Dispositif de mesure acoustique (1) selon l'une quelconque des revendications précédentes, dans lequel,

   - ledit au moins un composant de régulation d'écoulement étant conçu et agencé à l'intérieur dudit ensemble de pompe à air (2) de sorte que lesdites deux pompes à membrane piézo-électriques (3a, 3b) soient capables de fonctionner de manière bidirectionnelle lors de l'application de ladite procédure de commande de pression d'air.

5. Dispositif de mesure acoustique (1) selon l'une quelconque des revendications précédentes, dans lequel

   - lesdites au moins deux pompes (3a, 3b) comprenant une première pompe (3a) et une seconde pompe (3b), dans lequel ladite première pompe (3a) et ladite seconde pompe (3b) étant polarisées dans des directions opposées l'une à l'autre,
   et/ou
   - lesdites au moins deux pompes (3a, 3b) sont formées comme des pompes à membrane piézo-électriques (3a, 3b),
   et/ou
   - ledit ensemble de pompe à air (2) étant conçu de sorte que lesdites au moins deux pompes (3a, 3b), de préférence des pompes à membrane piézo-électriques (3a, 3b), puissent fonctionner simultanément pour commander ledit niveau de pression d'air dans ladite procédure de commande de pression d'air dans ledit canal auditif.

6. Dispositif de mesure acoustique (1) selon la revendication 5, dans lequel

   - lesdites au moins deux pompes (3a, 3b) sont formées comme des pompes à membrane piézo-électriques (3a, 3b) qui sont chacune agencées fluidiquement en série avec au moins un composant de régulation d'écoulement (4a, 4b), en particulier sous la forme d'une résistance d'écoulement (4a, 4b), dans lequel les deux pompes à membrane piézo-électriques (3a, 3b) sont agencées en parallèle l'une à l'autre.

7. Dispositif de mesure acoustique (1) selon la revendication 6, dans lequel

   - ledit ensemble de pompe à air (2) fournissant une première pompe à membrane piézo-électrique (3a) fluidiquement en série avec une première résistance d'écoulement (4a) et une seconde pompe à membrane piézo-électrique (3b) fluidiquement en série avec une seconde résistance d'écoulement (4b), dans lequel ladite première pompe à membrane piézo-électrique (3a) et ladite seconde pompe à membrane piézo-électrique (3b) sont agencées en parallèle l'une à l'autre,
   dans lequel
   - ledit ensemble de pompe à air (2) étant conçu et agencé de sorte que la pression entre ladite première résistance d'écoulement (4a) et ladite seconde résistance d'écoulement (4b) puisse être commandée dans ledit conduit auditif de ladite oreille,
   - ledit ensemble de pompe à air (2) agissant ainsi en particulier essentiellement de manière analogue à un diviseur de tension, en maintenant et en ajustant une pression d'entrée et une pression de sortie au niveau de ladite première (3a) et de ladite seconde pompe à membrane piézo-électrique (3b).

8. Dispositif de mesure acoustique (1) selon l'une quelconque des revendications 3 à 7, dans lequel

   - un silencieux acoustique est prévu et disposé au niveau de ladite sonde auriculaire,
   - ledit silencieux acoustique étant conçu pour constituer lesdits composants de régulation d'écoulement, en particulier sous la forme d'une résistance d'écoulement, et conçu pour atténuer le bruit provenant dudit ensemble de pompe à air (2),
   - ledit silencieux acoustique étant en particulier constitué d'un réseau de tubes et de cavités étroits, conçus pour atténuer le bruit acoustique et agissant à la manière d'une résistance d'écoulement.

9. Dispositif de mesure acoustique (1) selon l'une quelconque des revendications précédentes, dans lequel

- ledit ensemble de pompe à air (2), dans ladite position de repos, étant conçu pour appliquer ladite procédure de commande de pression d'air sous la forme du maintien essentiellement d'un niveau de pression d'air unique pendant ladite procédure, dans lequel
- ledit niveau de pression d'air étant compris dans la plage allant de -600 à +400 daPa, et/ou
- ledit ensemble de pompe à air (2), dans ladite position de repos, étant conçu pour appliquer ladite procédure de commande de pression d'air sous la forme de l'exécution de niveaux de pression d'air variables pendant ladite procédure, dans lequel lesdits niveaux de pression d'air variables pendant ladite procédure se situent entre environ -600 daPa et +400 daPa, dans lequel
- en particulier, ledit ensemble de pompe à air (2), dans ladite position de repos, étant conçu pour effectuer lesdits niveaux de pression d'air variables avec un débit de balayage de pression d'air réglable.

10. Dispositif de mesure acoustique (1) selon l'une quelconque des revendications précédentes, dans lequel

- ledit dispositif de mesure acoustique (1) étant conçu pour fournir des valeurs de propriétés acoustiques représentatives de l'oreille moyenne dudit patient en réaction à une première procédure de commande de pression de ladite procédure de commande de pression d'air, appliquée par lesdites au moins deux pompes (3a, 3b) dudit ensemble de pompe à air (2) à travers la membrane tympanique de ladite oreille dudit patient, dans lequel
- ladite première procédure de commande de pression contient en particulier l'exécution de niveaux de pression d'air variables pendant ladite procédure, dans lequel lesdits niveaux de pression d'air variables pendant ladite première procédure de commande de pression se situent entre environ - 600 daPa et +400 daPa, dans lequel
- en particulier, ledit ensemble de pompe à air (2), dans ladite position de repos, étant conçu pour effectuer lesdits niveaux de pression d'air variables avec un débit de balayage de pression d'air réglable,
- ledit dispositif de mesure acoustique (1) étant en particulier conçu pour agir comme un tympanomètre.

11. Dispositif de mesure acoustique (1) selon l'une quelconque des revendications précédentes, dans lequel

- ledit dispositif de mesure acoustique (1) étant conçu pour fournir des valeurs de propriétés acoustiques représentatives de l'oreille interne dudit patient en réaction à une deuxième procédure de commande de pression de ladite procédure de commande de pression d'air, appliquée par lesdites au moins deux pompes dudit ensemble de pompe à air (2) à travers la membrane tympanique de ladite oreille dudit patient, dans lequel
- ladite deuxième procédure de commande de pression contient en particulier l'exécution et le maintien essentiellement d'un niveau de pression d'air unique pendant ladite procédure, dans lequel
- ledit niveau de pression d'air essentiellement unique effectué étant en particulier compris dans la plage allant de - 600 à + 400 daPa,
- ledit dispositif de mesure acoustique (1) étant en particulier conçu pour agir comme un instrument de diagnostic auditif pour la mesure acoustique-réflexe sous pression.

12. Dispositif de mesure acoustique (1) selon l'une quelconque des revendications précédentes, dans lequel

- ledit dispositif de mesure acoustique (1) étant conçu pour fournir des valeurs de propriétés acoustiques représentatives de l'oreille interne dudit patient en réaction à une troisième procédure de commande de pression de ladite procédure de commande de pression d'air, appliquée par lesdites au moins deux pompes (3a, 3b) dudit ensemble de pompe à air (2) à travers la membrane tympanique de ladite oreille dudit patient, dans lequel
- ladite troisième procédure de commande de pression contient en particulier l'exécution et le maintien essentiellement d'un niveau de pression d'air unique pendant ladite procédure, dans lequel
- ledit niveau de pression d'air essentiellement unique effectué étant en particulier compris dans la plage allant de -600 à + 400 daPa,
- ledit dispositif de mesure acoustique (1) étant en particulier conçu pour agir comme un instrument de diagnostic auditif pour la mesure d'émission otoacoustique.

13. Dispositif de mesure acoustique (1) selon l'une quelconque des revendications précédentes, dans lequel le dispositif de mesure acoustique comprend un dispositif de commande conçu pour commander ledit ensemble de pompe à air (2).

14. Ensemble instrumental de diagnostic conçu pour fournir au moins un paramètre objectif de l'état de l'oreille d'un patient, en particulier de l'oreille d'un humain, comprenant

   - un dispositif de mesure acoustique (1) selon l'une quelconque des revendications 1 à 13,
   - un dispositif de traitement conçu pour traiter davantage lesdits signaux acoustiques capturés sur un dispositif d'évaluation, ledit dispositif d'évaluation étant conçu pour exécuter un processus d'évaluation desdits signaux acoustiques traités associés à ladite procédure de commande de pression d'air pour obtenir ledit au moins un paramètre objectif de l'état de l'oreille pour ladite oreille du patient.

15. Ensemble instrumental de diagnostic selon la revendication 14, dans lequel ledit ensemble instrumental de diagnostic peut être connecté mécaniquement à un dispositif autonome et/ou être actionné via un dispositif d'extrémité mobile, en particulier une tablette ou un smartphone.

16. Procédé utilisant un dispositif de mesure acoustique (1) selon l'une quelconque des revendications 1 à 13 et/ou un ensemble instrumental de diagnostic selon l'une quelconque des revendications 14 ou 15, le procédé comprenant

   - le positionnement d'au moins une partie du dispositif de mesure acoustique (1) à l'intérieur d'un conduit auditif d'un patient pour prendre une position de repos à l'intérieur dudit conduit auditif,
   - la fourniture d'une connexion fluidique entre un ensemble de pompe à air (2) dudit dispositif de mesure acoustique (1), au moins dans ladite position de repos, et ledit conduit auditif à travers au moins un tube passant par une sonde auriculaire dudit dispositif de mesure acoustique (1),
   - l'application d'une procédure de commande de pression d'air, constituée d'au moins un niveau de pression d'air, à travers la membrane tympanique de ladite oreille dudit patient, par l'ensemble de pompe à air (2), dans ladite position de repos, et
   - la fourniture de valeurs de propriétés acoustiques en réaction à ladite procédure de commande de pression d'air appliquée par ledit ensemble de pompe à air (2) à travers la membrane tympanique de ladite oreille dudit patient, par le dispositif de mesure acoustique (1), dans ladite position de repos.

FIG. 1

FIG. 2

FIG. 3

FIG. 4

**EP 4 295 766 B1**

REFERENCES CITED IN THE DESCRIPTION

**Patent documents cited in the description**

- US 2016220155 A1
- US 2015342504 A1